Europäisches Patentamt

European Patent Office

Office européen des brevets

(11) Publication number: **0 319 314**
**A2**

# EUROPEAN PATENT APPLICATION

(21) Application number: **88311433.2**

(22) Date of filing: **02.12.88**

(51) Int. Cl.⁴: **A 61 F 13/16**

(30) Priority: **03.12.87 US 128364**

(43) Date of publication of application:
**07.06.89 Bulletin 89/23**

(84) Designated Contracting States:
**AT BE CH DE ES FR GB GR IT LI LU NL SE**

(71) Applicant: **THE PROCTER & GAMBLE COMPANY**
**One Procter & Gamble Plaza**
**Cincinnati Ohio 45202 (US)**

(72) Inventor: **Litchholt, John Joseh**
**101 Hopping Court**
**Harrison Ohio 45030 (US)**

(74) Representative: **Gibson, Tony Nicholas et al**
**Procter & Gamble (NTC) Limited Whitley Road**
**Longbenton Newcastle upon Tyne NE12 9TS (GB)**

(54) Dual function disposable absorbent insert having a removable backsheet.

(57) A dual function disposable absorbent insert having an absorbent element and a removable liquid impervious backsheet is provided. Upon removal of the backsheet, the absorbent element retains structural integrity and allows liquids to pass clear through. In a preferred embodiment, the insert comprises (from the body contacting layer down): a liquid permeable topsheet, an absorbent core, a liquid permeable bottomsheet, and a liquid impermeable backsheet. The backsheet is removable. In its four-layer form the insert can be placed in a user's underpants to provide both absorption and containment of body exudates. With the backsheet removed and with the insert in its three layer form, the insert is intended to be used as a secondary absorbent structure which is placed inside of a primary absorbent structure such as a diaper or brief. In this form the insert acts as an absorbent booster for the primary absorbent structure by providing quick acquisition of liquid gushes and greater total absorbency.

**Description**

## DUAL FUNCTION DISPOSABLE ABSORBENT INSERT HAVING A REMOVABLE BACKSHEET

### FIELD OF THE INVENTION

The present invention relates to integral disposable absorbent articles such as disposable inserts, and in particular to dual function disposable inserts which can function as primary absorbent and containment articles or which upon removal of their liquid impermeable backsheets can function as secondary absorbent articles.

### BACKGROUND OF THE INVENTION

Articles of the general type described are well known. In particular, disposable inserts are widely described in the patent literature and elsewhere. For example, U.S. Patent 3,886,941, which issued to Duane et al. on June 3, 1975, discloses a disposable diaper insert comprising an absorbent pad disposed between a liquid permeable hydrophobic topsheet and a liquid impermeable bottomsheet, each of said sheets having a plurality of valvular openings therein.

In addition, disposable diapers, incontinent garments and sanitary napkins having removable layers are widely described in the patent literature and elsewhere. For example, U.S. Patent 4,417,894, which issued to Norris on November 29, 1983, discloses a disposable diaper containing a towelsheet superposed on or above the backsheet of the diaper. When the diaper is soiled, the towelsheet may be unfastened at the bottom area of the diaper and used as a towel to clean solid waste from the child during the diaper removal process. U.S. Patent 4,327,729, which issued to King on May 4, 1982, and U.S. Patent 3,952,745, which issued to Duncan on April 27, 1976, disclose disposable diapers having removable liquid impervious backsheets which are removed when soiled to permit easy flushing of the remainder of the diaper. In each case, the absorbent layer is contained between layers of wet strength tissue paper, the lowermost layer of wet strength tissue being adhered to the backsheet. The backsheet is secured to the topsheet along the edges of the diaper by glue beads. When the diaper is soiled, the backsheet is pulled away from the diaper thus severing the bonds between the topsheet and backsheet but not the bonds between the wet strength tissue and backsheet. Thus, the backsheet and a large central panel of the wet strength tissue are removed from the diaper, thus exposing the absorbent pad. Such exposure facilitates hydraulic erosion of the absorbent pad in a toilet bowl for ease of flushability.

U.S. Patent 3,874,385, which issued to Gellert on April 1, 1975, and U.S. Patent 3,842,838, which issued to Gellert on October 22, 1974, disclose disposable diapers having removable topsheets which are removed for easy disposal of solid wastes. Number 3,874,385, discloses a disposable diaper in which the entire topsheet can be peeled away along its edges from the remainder of the diaper. Number 3,842,838, discloses a disposable diaper in which a portion of the topsheet is perforated so that when soiled, that portion can be torn away from the remainder of the diaper.

From the foregoing, it is clear that absorbent articles having removable layers, and especially removable backsheets, are not new. However, what is new and unique about the present invention, unlike the prior articles having removable backsheets, is that the present invention retains its structural integrity upon removal of the backsheet. In other words, the prior art articles were designed so that when the backsheet was stripped away, the tissue layer that encased the airfelt absorbent core was also stripped away. Thus, the airfelt core was left unwrapped and unencased making it easy for it to erode, disperse, break up or pull apart. The present invention is designed so that when the backsheet is stripped away, the absorbent element will maintain structural integrity.

In summary, the prior art does not disclose a dual function disposable absorbent article, and more particularly a dual function disposable absorbent insert, which for low-flow (low void volume) users can function as a primary absorbent structure and which for high-flow (high void volume) users can function as a secondary absorbent structure, absorbent "booster", or diaper or incontinent brief insert.

Therefore, it is an object of the present invention to provide a disposable absorbent article which can function as both a primary absorbent structure and as a secondary absorbent structure. It is a further object of the present invention to provide a disposable absorbent article comprising an absorbent element having structural integrity and a removable liquid impermeable backsheet which prevents liquids from passing completely through said article, and whereupon removal of the backsheet the absorbent element maintains its structural integrity while allowing liquids to pass completely through said article.

### SUMMARY OF THE INVENTION

In accordance with the present invention, a dual function disposable absorbent article, and more particularly a dual function disposable absorbent insert, having a removable liquid impervious backsheet is provided. Preferably, the insert comprises four layers. From the body contacting layer down the four layers are a liquid permeable topsheet, an absorbent core, a liquid permeable bottomsheet, and a liquid impermeable backsheet. The backsheet is removable. For low-flow (low void volume) users, the insert can be placed in the user's underpants in its four layer form. In this four layer form the insert provides both absorption and containment and prevents absorbed exudates from contacting and soiling other garments or linens. For high-flow (high void volume) users, the liquid impermeable backsheet may be removed and the insert placed inside of a primary absorbent structure such as a diaper or brief. In this three layer form the insert provides absorption but may not completely contain absorbed exudates nor prevent them from contacting and soiling other garments and linens. In this three layer form the insert is intended to act as an absorbent booster for the primary absorbent structure by providing quick acquisition of liquid gushes and greater total absorbency.

## BRIEF DESCRIPTION OF THE DRAWINGS

While the specification concludes with claims particularly pointing out and distinctly claiming the subject matter which is regarded as forming the present invention, it is believed that the invention will better understood from the following descriptions which are taken in conjunction with the accompanying drawings in which like designations are used to designate substantially identical elements and in which:

Figure 1 is a top plan view of a disposable insert embodiment of the present invention having portions cut away to reveal underlying structure and with the portion of the insert which contacts the wearer facing the viewer.

Figure 2 is an enlarged transverse cross-sectional view of the disposable insert embodiment shown in Figure 1 taken along section line 2-2 of Figure 1.

Figure 3 is an enlarged longitudinal cross-sectional view of the disposable insert embodiment shown in Figure 1 taken along section line 3-3 of Figure 1.

Figure 4 is a bottom plan view of an alternatively preferred disposable insert embodiment of the present invention shown without any hidden lines and with the portion of the insert which contacts the wearer's garments facing the viewer.

Figure 5 is an enlarged inverted transverse cross-sectional view of the disposable insert embodiment shown in Figure 4 taken along section line 5-5 of Figure 4.

Figure 6 is a bottom plan view of another alternatively preferred disposable insert embodiment of the present invention shown without any hidden lines and with the portion of the insert which contacts the wearer's garments facing the viewer.

Figure 7 is an enlarged transverse cross-sectional view of another alternatively preferred disposable insert embodiment of the present invention.

## DETAILED DESCRIPTION OF PREFERRED EMBODIMENTS

The present invention relates to integral disposable absorbent articles such as disposable inserts, and more particularly, to dual function disposable absorbent inserts having a removable liquid impermeable backsheet.

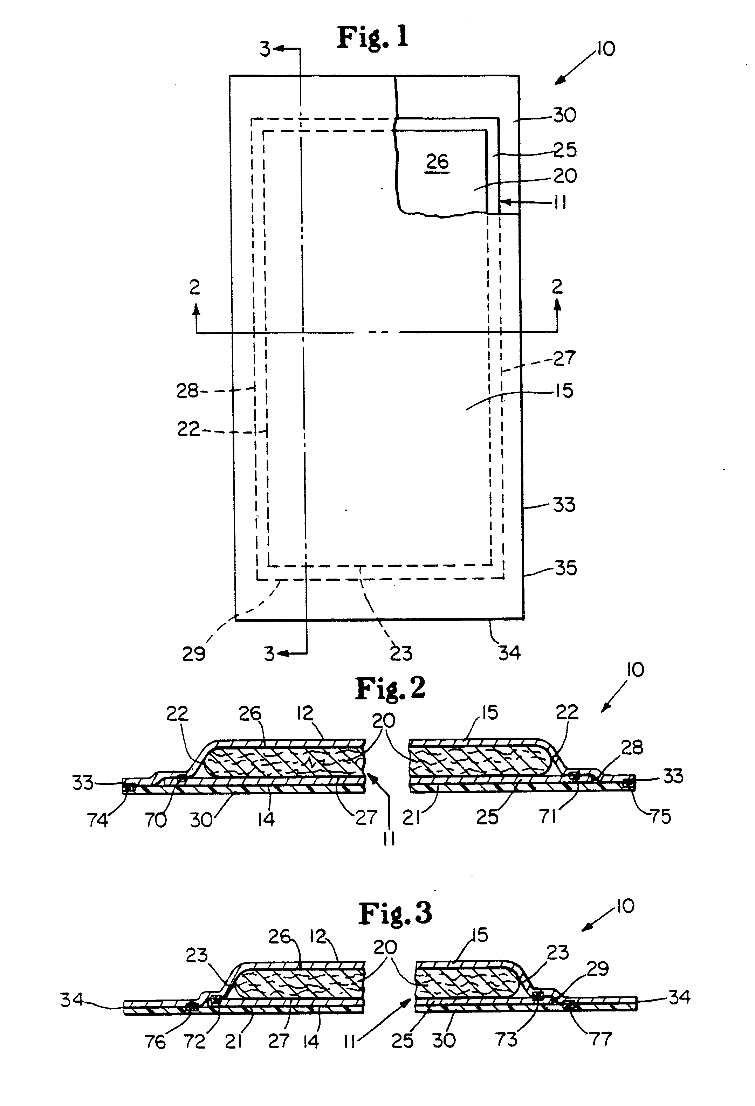

As used herein, the term "integral disposable absorbent article" refers to articles which absorb and contain body exudates and more specifically refers to articles which are placed against or in proximity to the body of a wearer to absorb and contain the various exudates discharged from the body and which are intended to be discarded after a single use (i.e., they are not intended to be laundered or otherwise restored or reused). A preferred embodiment of the integral disposable absorbent article of the present invention is shown in Figure 1 as it would be used as an insert 10. As used herein, the term "insert" refers to an article which is placed inside a garment that has been suspended about the lower torso of a wearer, such as underwear, pants, shorts, diapers, or incontinent briefs.

Figure 1 is a top plan view of the insert 10 with portions of the structure being cut-away to more clearly show the construction of the insert 10 and with the portion of the insert 10 which contacts the wearer facing the viewer. Figure 2 is a transverse cross-sectional view of the insert 10 taken along line 2-2 of Figure 1. Figure 3 is a longitudinal cross-sectional view of the insert 10 taken along line 3-3 of Figure 1. In the preferred embodiment shown in Figures 1, 2, and 3, the insert 10 basically comprises an absorbent element 11 and a removable liquid impermeable backsheet 30. The absorbent element 11 has a body surface 12 and a garment surface 14. The backsheet 30 overlays the garment surface 14 of the absorbent element 11 and is removably affixed to the absorbent element 11. As used herein, the term "removable" is intended to encompass configurations whereby the entire backsheet 30, or only a lesser portion 45 of the backsheet 30, is separable from the remainder of the absorbent article without destroying the structural integrity of the absorbent element 11. The absorbent element 11 may be any means which has structural integrity and which is generally compressible, conformable, non-irritating to the wearer's skin and capable of absorbing body exudates such as feces, urine, blood, pus, and the like. As used herein, and throughout this specification, the term "structural integrity" refers to the characteristic of the absorbent element 11 of not being subject to dispersion, erosion, breaking up or pulling apart, either due to the absorbent element's 11 own physical characteristics or due to the fact that a member of the absorbent element 11, which otherwise might be subject to dispersion, erosion, breaking up or pulling apart, is prevented from so doing by being contained within other members of the absorbent article. In the preferred embodiment, the absorbent element 11 comprises a liquid permeable topsheet 15, an absorbent core 20 and a liquid permeable bottomsheet 25. The absorbent core 20 has a first major surface 26, a second major surface 27, core side edges 22 and core waist edges 23. Preferably, the topsheet 15 and the bottomsheet 25 extend beyond the edges 22 and 23 of the absorbent core 20 where they are affixed together in a suitable manner. As used herein, the term "affixed" encompasses configurations whereby a first member is directly joined to a second member by affixing the first member directly to the second member and configurations whereby the first member is indirectly joined to the second member by affixing the first member to intermediate members which in turn are affixed to the second member. Also, preferably, the topsheet 15 and the backsheet 30 extend beyond the bottomsheet side edges 28 and the bottomsheet end edges 29 where they are affixed together in a suitable manner. In the preferred embodiment shown in Figures 1, 2 and 3, the extension of the topsheet 15 and the backsheet 30 beyond bottomsheet side edges 28 and the bottomsheet end edges 29 forms the longitudinal edges 33 and the end edges 34, respectively, of the insert 10. The longitudinal edges 33 and the end edges 34 comprise the periphery 35. In this four layer form, with the backsheet 30 affixed to the remainder of the insert 10, the insert 10 is primarily intended for use as a primary absorbent structure. As used herein, the term "primary absorbent structure" refers to an absorbent article which provides both absorption and containment of exudates. Examples include disposable diapers, incontinent briefs, and other articles commonly characterized by having a liquid impermeable backsheet.

When the insert 10 is intended for use a secondary absorbent structure which is to be placed inside of a primary absorbent structure, the backsheet 30 is removed from the remainder of the insert 10 before the insert 10 is used. Thus, the insert 10, which in its original preferred embodiment had four layers, is transformed into a three layer structure. As used herein, the term "secondary absorbent structure" refers to an absorbent article which will provide absorption but may not provide complete containment of liquid exudates. Examples include disposable inserts not having a liquid impermeable backing. In this form the insert 10 comprises a liquid permeable topsheet 15, an absorbent core 20 and a liquid permeable bottomsheet 25. In this form the insert 10 allows liquids to pass completely therethrough. Therefore, in this form the insert 10 is intended to be used as a secondary absorbent structure which is placed inside of a primary absorbent structure such as a diaper or incontinent brief and which acts as an absorbent booster for quick acquisition of liquid gushes and which provides the primary absorbent structure with a greater liquid absorbent capacity.

Examining some of the elements of the insert 10 in more detail, the topsheet 15 is positioned adjacent the first major surface 26 of the absorbent core 20 and overlays a major portion of the absorbent core 20, so that when exudates are discharged onto the topsheet 15 they penetrate through the topsheet 15 where they are absorbed by the absorbent core 20. The topsheet 15 is compliant, soft feeling, and non-irritating to the wearer's skin. Further, the topsheet 15 is liquid permeable, permitting liquids to readily penetrate through its thickness. A suitable topsheet may be manufactured from a wide range of materials such as porous foams, reticulated foams, apertured plastic films, natural fibers (e.g., wood or cotton fibers), synthetic fibers (e.g., polyester or polypropylene fibers) or from a combination of natural and synthetic fibers, with formed films being preferred. Preferably it is made of a hydrophobic material to isolate the wearer's skin from liquids in the absorbent core 20. The formed films are preferred for the topsheet 15 because they are pervious to liquids and yet non-absorbent. Thus, the surface of the formed film, which is in contact with the body, remains dry, thereby reducing body soiling and creating a more comfortable feel for the wearer. Suitable formed films are described in U.S. Patent No. 3,929,135, entitled "Absorptive Structure Having Tapered Capillaries", which patent issued to Thompson on December 30, 1975, U.S. Patent No. 4,324,246, entitled "Disposable Absorbent Article Having a Stain Resistant Topsheet", which patent issued to Mullane and Smith on April 13, 1982, U.S. Patent No. 4,342,314, entitled "Resilient Plastic Web Exhibiting Fiber-Like Properties", which patent issued to Radel and Thompson on August 3, 1982, and U.S. Patent No. 4,463,045, entitled "Macroscopically Expanded Three-Dimensional Plastic Web Exhibiting Non-Glossy Visible Surface and Cloth-Like Tactile Impression", which patent issued to Ahr, Louis, Mullane, and Ouellette on July 31, 1984,

A particularly preferred topsheet 15 comprises staple length polypropylene fibers such as Hercules Type 151 polypropylene marketed by Hercules, Inc. of Wilmington, Delaware. As used herein, the term "staple length fibers" refers to those fibers having a length of at least 15.9 millimeters (0.625 inches).

There are a number of manufacturing techniques which may be used to manufacture the topsheet 15. For example, the topsheet 15 may be woven, non-woven, spunbonded, carded, or the like. A preferred topsheet 15 is carded and thermally bonded by means well known to those skilled in the fabrics art. Preferably, the topsheet 15 has a weight of from about 18 to about 25 grams per square meter, a minimum dry tensile strength of at least about 400 grams per centimeter in the machine direction and a wet tensile strength of at least about 55 grams per centimeter in the cross-machine direction.

The absorbent core 20 may be any means which is generally compressible, conformable, non-irritating to the wearer's skin, and capable of absorbing and containing liquids and certain body exudates. The absorbent core 20 may be manufactured in a wide variety of sizes and shapes (e.g., rectangular, hourglass, etc.) and from a wide variety of liquid absorbent materials commonly used in disposable diapers and other absorbent articles, such as comminuted wood pulp which is generally referred to as airfelt. Examples of other suitable absorbent materials include creped cellulose wadding, absorbent foams, absorbent sponges, superabsorbent polymers, absorbent gelling materials, or any equivalent materials or combination of materials. The total absorbent capacity of the absorbent core 20 should, however, be compatible with the design exudate loading for the intended use of the insert 10. Further, the size and absorbent capacity of the absorbent core 20 may be varied to accommodate wearers ranging from infants to adults.

A preferred embodiment of the insert 10 has a rectangular shaped absorbent core 20 and is intended to be worn by adults. The absorbent core 20 is preferably a batt of airfelt 10.0 centimeters (4.0 inches) wide (lateral dimension) and 21.0 centimeters (8.4 inches) long (longitudinal dimension). The airfelt use in the absorbent core 20 has a generally uniform caliper of 1.0 centimeter (0.40 inch), an absorbent capacity of from 8 grams to 16 grams of water per gram of absorbent material, and a weight of from 0.03 grams per cubic centimeter to 0.07 grams per cubic centimeter. It should be understood, however, that the size, shape, configuration, and total absorbent capacity of the absorbent core 20 may be varied to accommodate wearers ranging from infants through adults. Therefore, the dimensions, shape, and configuration of the absorbent core 20 may be varied (e.g., the absorbent core may have a varying caliper, or a hydrophilic gradient or may contain absorbent gelling materials).

The bottomsheet 25 is positioned adjacent the second major surface 27 of the absorbent core 20 and overlays a major portion of the absorbent core 20. The bottomsheet 25 is liquid permeable, permitting liquids to readily penetrate through its thickness. While a number of materials and manufacturing techniques may be used to manufacture the bottomsheet 25, satisfactory results have been obtained with sheets of tissue paper having a basis weight of 16 grams per square meter (10 pounds per 3,000 square feet) and having an air permeability of 30.5 cubic meters per minute per square meter (100 cubic feet per minute per square foot) at a pressure differential of 12.8 millimeters of water (0.5 inch). Preferably, the bottomsheet 25 possesses a number of the beneficial features of the topsheet 15, such as being compliant, soft feeling, and non-irritating to the wearer's skin. This is preferable because if the backsheet 30 is removed from the remainder of the insert 10, the insert 10 may be placed, either purposely or inadvertently, in a primary absorbent structure with the bottomsheet 25 facing upwards towards the wearer's skin. In a preferred embodiment, the bottomsheet 25 and the topsheet 15 are manufactured from the same materials. One preferred bottomsheet 25 is a formed film. Suitable formed films are those described earlier in relation to the topsheet 15. In some embodiments of the present invention, the backsheet 30 is affixed to the bottomsheet 25 and, therefore, the bottomsheet 25 may have to resist some tensile forces if the backsheet 30 is stripped away from the insert 10. In such embodiments, the bottomsheet 25 preferably has a dry tensile strength in both the machine and cross-directions of greater than 70 grams per centimeter, more preferably greater than 100 grams per centimeter, more preferably greater than 160 grams per centimeter, and most preferably greater than 320 grams per centimeter. Also, preferably, the bottomsheet 25 has a wet tensile strength in both the machine and cross-directions of greater than 10 grams per centimeter, more preferably greater than about 20 grams per centimeter, more preferably greater than about 80 grams per centimeter, and most preferably greater than about 160 grams per centimeter.

Tensile strength is determined using a tensile tester constructed in such a way that a gradually increasing load is smoothly applied to a defined sample portion until the sample portion fails. Examples of such tensile testers are Instron 1101-TM, 1102-TMS, 1122 or 1130, made by the Instron Engineering Corporation, Canton, Massachusetts, or the Thwing-Albert Intellect 500 made by the Thwing-Albert Instrument Company, Philadelphia, Pennsylvania. Results are reported as the average load in force per sample width at failure. The test is performed in a room controlled to 22.8 ± 1.1°C (73 ± 2°F) and 50 ± 2% relative humidity. Four 2.5 centimeters x 17.5 centimeters (1.0 inch x 7.0 inches) samples are conditioned in the room for a minimum of two hours. The tensile tester is calibrated and zeroed according to A.M.I.-L-3 and the manufacturer's instructions. Set the instrument crosshead to operate at 25.0 centimeters (10.0 inches) per minute. Set the gauge length at 12.5 centimeters (5.0 inches) for machine direction or 5.0 centimeters (2.0 inches) for cross direction. Insert one sample strip into the top clamp. Align the strip between the top and bottom clamps and clamp the strip into the bottom clamp with enough tensile to eliminate any slack in the sample. Start the tensile tester as described in the manufacturer's instructions for the particular tester used. Continue the test until the crosshead automatically returns to its initial starting position or until the recorder pen returns to zero. Read and record the tensile peak load from the digital panel meter or the stress strain curve to the nearest unit.

$$\frac{\text{Average Tensile (grams)}}{\text{(inch width)}} = \frac{\text{(sum of peak loads)}}{\text{(number of sample strips tested)}}$$

Wet tensile strength is also determined using a tensile tester constructed in such a way that a gradually increasing load is smoothly applied to a defined sample portion until the sample portion fails. The same tensile testers as used in the dry tensile strength test can also be used for the wet tensile test. In addition, a Finch Wet Strength Device, obtained from the Thwing-Albert Instrument Company, consisting of an inverted stirrup about 3.75 centimeters (1.5 inches) in width and 7.5 centimeters (3.0 inches) in length, made of a metal strap by which a horizontal rod, 2.75 centimeters (1.1 inches) in length and 0.47 ± 0.055 centimeter (0.188 ± 0.022 inch) in diameter, is supported, or a similar device, is needed. Between the straps and under the horizontal rod is a small, vertically movable container for holding water or other liquid. The liquid container locks in its uppermost position so that the horizontal rod is then immersed in the liquid to a depth of at least 1.9 centimeters (0.75 inch). A thin metal tang forming the lower part of the inverted stirrup permits it to be fastened in the lower clamp of a tension testing machine. A modified design using a plastic cup is acceptable providing a basic operation similar to that described is used. The test is performed in a room controlled to 22.8 ± 1.1°C (73 ± 2°F) and 50 ± 2% relative humidity. The tensile tester is calibrated and zeroed according to A.M.I.-L-3 and the manufacturer's instructions. Operate the tensile tester at a crosshead speed of 10.0 centimeters (4.0 inches) per minute. Fasten the Finch Wet Strength Device in the lower clamp of the tensile tester so that the horizontal rod is parallel to the clamp faces and is otherwise symmetrically located with respect to the clamps. Adjust the position of the lower clamp so that the horizontal axis of the rod is exactly the distance below the upper clamp required depending upon direction being tested; 10.0 centimeters (4.0 inches) for machine direction or 5.0 centimeters (2.0 inches) for cross direction. Move the liquid container to its lowest position and fill to 0.31 centimeters (0.125 inch) from the top of the container with distilled water. Four 25.0 centimeters x 2.5 centimeters (10.0 inches x 1.0 inch) samples are conditioned in the room for a minimum of two hours. Thread one of the sample strips prepared for testing under the rod in the Finch Wet Strength Device. Place the ends of the specimen together, remove the slack, and fasten them in the upper clamp of the tensile tester machine. The specimen should be centrally located with respect to the horizontal rod and the upper clamp. Raise the liquid container so that it locks in its uppermost position, immersing the looped end of the specimen to a depth of at least 1.88 centimeters (0.75 inch). Exactly five seconds after the liquid container is raised in place and with the liquid container remaining in place, engage the tensile tester. Repeat the above procedure on the other 3 strips prepared.

$$\text{Wet Tensile} \left( \frac{\text{grams}}{\text{(inch width)}} \right) = \frac{\text{(sum of loads at break for strips tested )}}{\text{(2 x total number of tensile strips tested)}}$$

NOTE: The two in the denominator results from each sample strip being, in essence, doubled to represent two strips under the conditions of this test. That is, each single strip extends between the top and bottom jaw twice.

The backsheet 30 is positioned adjacent to the bottom surface 21 of the bottomsheet 25 which is the side of the bottomsheet 25 furthest away from the absorbent core 20. The backsheet 30 is impermeable to liquids and is preferably manufactured from a thin plastic film although other flexible liquid impermeable materials may also be used. The backsheet 30 prevents the exudates absorbed and contained in the absorbent core 20 from wetting articles which contact the insert 10, such as bedsheets and undergarments. Preferably, the backsheet 30 is a polyethylene film having a thickness of from 0.012 millimeters (0.5 mil) to 0.051 centimeters (2.0 mils), although other flexible, liquid impermeable materials may be used. As used herein, the term "flexible" refers to materials which are compliant and which will readily conform to the general shape and contours of the human body.

A suitable polyethylene film is manufactured by Monsanto Chemical Corporation and marketed in the trade as film No. 8020. The backsheet 30 is preferably embossed and/or matte finished to provide a more clothlike appearance. Further, the backsheet 30 may permit vapors to escape from the absorbent core 20 while still preventing exudates from passing through the backsheet 30.

The backsheet 30 is removable from the remainder of the insert 10. The essence of this invention lies in the fact that a single absorbent insert can serve two purposes. When the backsheet 30 remains attached to the insert 10, as illustrated in the four layer insert 10 of Figures 1, 2 and 3, the insert 10 serves as an independent integral disposable absorbent article which provides both absorbency and containment of body exudates and which prevents the absorbed exudates from wetting articles which contact the insert 10. Therefore, in this form, the insert 10 would seem to be most beneficial as an independent or primary absorbent structure which could be placed directly into a wearer's underpants and which would function adequately without the need for another absorbent structure to be used in conjunction therewith.

Conversely, upon removal of the liquid impervious backsheet 30, the insert 10 still provides absorbency, but may not completely contain all the absorbed exudates nor prevent them from wetting articles which contact the insert 10. In this three layer form the insert 10 would seem to be most beneficial as a secondary absorbent structure which is placed inside of a primary absorbent structure such as a diaper or an incontinent brief so as to provide quick acquisition of liquid gushes and greater total absorbency.

The manner in which the various elements in the preferred embodiment of the insert 10 are assembled is clearly illustrated in Figures 1, 2 and 3. As seen in Figures 1, 2 and 3, the topsheet 15 overlays the first major surface 26 of the absorbent core 20 and extends beyond the core side edges 22 and the core end edges 23 to the longitudinal edges 33 and the end edges 34, respectively, of the insert 10. The topsheet 15 may, but need not be, affixed to the absorbent core 20. Examples of ways in which the topsheet 15 might be affixed to the absorbent core 20 are by spray gluing the topsheet 15 to the first major surface 26 of the absorbent core 20, by applying lines or spots of adhesive glue beads between the topsheet 15 and the first major surface 26 of the absorbent core 20, or by affixing the topsheet 15 along the core side edges 22 and the core end edges 23 by lines or spots of adhesive glue beads. The bottomsheet 25 overlays the second major surface 27 of the absorbent core 20 and extends beyond the core side edges 22 and the core end edges 23. However, in the preferred embodiment, the bottomsheet 25, unlike the topsheet 15 and the backsheet 30, does not extend to the longitudinal edges 33 and the end edges 34 of the insert 10. The overlapping portions of the topsheet 15 and the bottomsheet 25 are affixed to each other near and along the bottomsheet side edges 28 and the bottomsheet end edges 29 by second attachment means, the second attachment means being nonfrangible and being well known in the art. The second attachment means may be, for example, a uniform continuous layer of adhesive, a patterned layer of adhesive, or an array of separate lines or spots of adhesive. As used herein, the term "frangible" refers to the capability of the bond which is provided by an attachment means between two adjacent layers to be broken, thus freeing the two layers from each other. The term "nonfrangible", therefore, refers to a bond which is not subject to breaking when the insert 10 is being used as intended. In this preferred embodiment, the second attachment means are lines of adhesive 70, 71, 72 and 73.

Although in the previous paragraph the construction of the backsheet 30 with the insert 10 was not yet described, what was described was the structural configuration the insert 10 will possess if the backsheet 30 is removed from the insert 10. From the foregoing, it is clear that that absorbent core 20 is contained within the topsheet 15 and the bottomsheet 25, and thus, the absorbent element 11, and particularly the absorbent core 20, maintains its structural integrity after the backsheet 30 is removed. In other words, the absorbent core 20 is not subject to dispersion, erosion, breaking up or pulling apart.

The backsheet 30 overlays the bottomsheet 25 and extends beyond the bottomsheet side edges 28 and the bottomsheet end edges 29 to the longitudinal edges 33 and the end edges 34, respectively of the insert 10. The backsheet 30 and the topsheet 15 are affixed to each other beyond the bottomsheet side edges 28 and the bottomsheet end edges 29 by first attachment means. In this preferred embodiment, the first attachment means are frangible lines of adhesive 74, 75, 76 and 77.

In the preferred embodiment, the lines of adhesive 74 and 75 are near, if not along, the longitudinal edges 33 of the insert 10 so as to reduce scrap and to prevent the longitudinal edges of the topsheet 15 and the backsheet 30 from separating. However, along the end edges 34 of the insert 10 it is preferable that the topsheet 15 and the backsheet 30 be separable enough that the user can separate the two sheets and grasp enough of the backsheet 30 to get a firm grip when stripping the backsheet 30 from the remainder of the insert 10. Therefore, along the ends of the insert 10, it is preferable that the lines of adhesive 76 and 77 be placed a somewhat greater distance in from the end edges 34 of the insert 10 than the lines of adhesive 74 and 75 are placed in from the longitudinal edges 33 of the insert 10.

Further, it is preferable that the lines of adhesive 74, 75, 76 and 77 have a tensile and shear strength less than the tensile and shear strengths of the topsheet 15 and the backsheet 30. Such is preferable so that when the backsheet 30 is stripped from the remainder of the insert 10, the bond of the lines of adhesive 74, 75, 76 and 77 will be broken before the backsheet 30 or the topsheet 15 tears.

The size of the backsheet 30 and/or the topsheet 15 and/or the bottomsheet 25 are dictated by the size of the absorbent core 20 and the exact insert design selected. In a preferred embodiment, the topsheet 15, the bottomsheet 25, and the backsheet 30 have a rectangular shape. The topsheet 15 and the backsheet 30 extend beyond the core side edges 22 and the core waist edges 23 of the absorbent core 20 a distance of 2.5 centimeters (1.0 inch) to 5.5 centimeters (2.2 inches), where they are joined directly to each other along the longitudinal edges 33 and the end edges 34 of the insert 10 by the lines of adhesive 74, 75, 76 and 77. The bottomsheet 25 extends beyond the core side edges 22 and the core end edges 23 of the absorbent core 20 a distance of 1.25 centimeters (0.5 inch) to about 3.75 centimeters (1.5 inches) where it is joined directly to the topsheet 15 along the bottomsheet side edges 28 and the bottomsheet end edges 29 by the lines of adhesive 70, 71, 72 and 73.

When it is necessary for a particular use that the backsheet 30 be removed from the remainder of the insert 10, the user simply grips the backsheet 30 along an end edge 34 in one hand and the topsheet 15 along the same end edge 34 in the other hand and pulls the two sheets apart thus breaking the bond of the frangible lines of adhesive 74, 75, 76 and 77 between the backsheet 30 and the topsheet 15. The nonfrangible lines of adhesive 70, 71, 72, and 73 between the topsheet 15 and the bottomsheet 25 are not broken and thus the absorbent core remains contained between the topsheet 15 and the bottomsheet 25. Consequently, the absorbent element 11 retains structural integrity.

In a particularly preferred embodiment of the present invention (not shown), the insert 10 has side flaps, one or more side flap elastic members associated with each of said side flaps, and a pair of gasketing cuffs. The side flaps comprise those portions of the insert between the core side edges and the longitudinal edges. The side flap elastic members are operatively associated with the side flaps so that they effectively contract or

7

gather the side flaps to provide gasketing cuffs about the legs of the wearer. While the topsheet, the absorbent core, the bottomsheet, the backsheet, the side flaps, the side flap elastic members, and the gasketing cuffs may be assembled in a variety of well known configurations, a preferred configuration is described generally in U.S. Patent 3,860,003, entitled "Contractible Side Portions for Disposable Diaper", which patent issued to K.B. Buell on January 14, 1975

In addition, a method and apparatus suitable for manufacturing a disposable absorbent insert having elastically contractible gasketing cuffs are described in U.S. Patent 4,081,301, entitled "Method and Apparatus for Continuously Attaching Discrete, Stretched Elastic Strands to Predetermined Isolated Portions of Disposable Absorbent Products", which patent issued to K.B. Buell on March 28, 1979

As would be obvious to a person of ordinary skill in the art, the insert 10 may take the form of a number of alternative embodiments. For example, the insert 10 could comprise as few as two different layers (i.e., an absorbent layer superimposed on a liquid impermeable layer) on up to an infinite number of different layers so long as the insert 10 can be transformed from an insert that prevents liquids from passing clear through to an insert that upon removal of a liquid impermeable sheet will allow liquids to pass clear through but yet will maintain structural integrity.

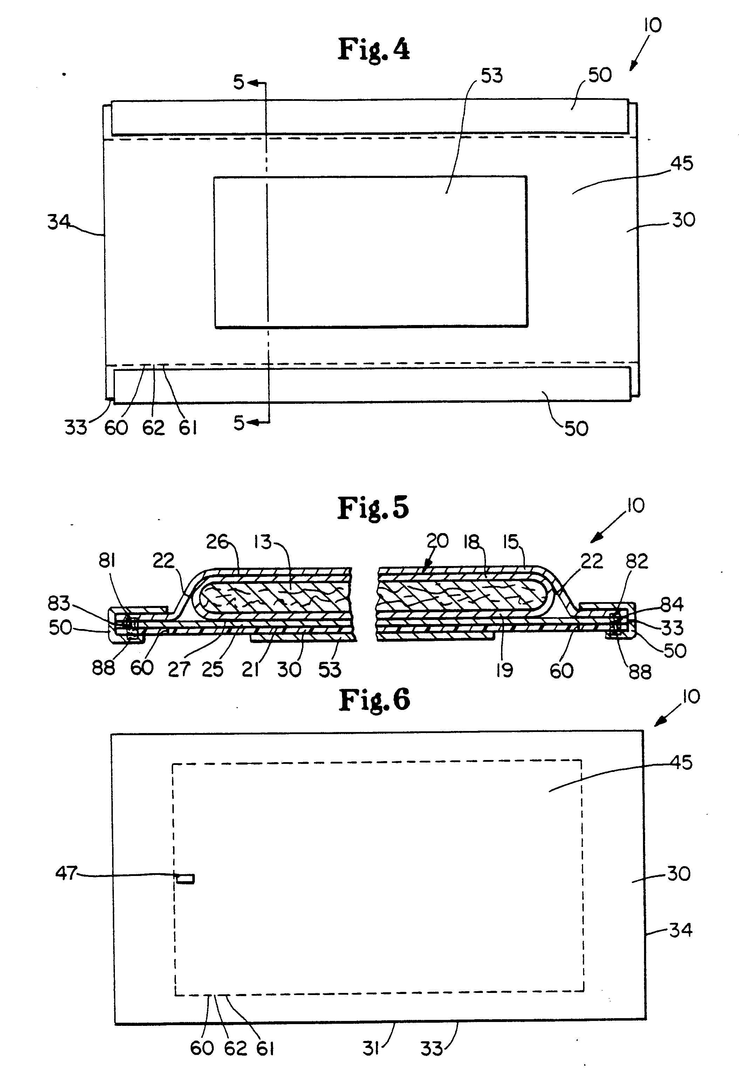

An alternatively preferred embodiment of the present invention is shown in Figures 4 and 5. Figure 4 is a bottom plan view of the insert 10 without any hidden lines and Figure 5 is an enlarged inverted cross-sectional view of the insert 10 taken along line 5-5 of Figure 4. In this embodiment, an alternatively preferred absorbent core 20 comprising an absorbent layer 13 which is enveloped between a first tissue layer 18 and a second tissue layer 19, is shown. The tissue layers 18 and 19 are utilized to provide in use core integrity and to prevent dusting or linting of the absorbent layer 13 through the porous, body contacting topsheet 15. This is especially true when the absorbent layer 13 is an airfelt layer. The bottom tissue layer 19 prevents dusting or linting through the bottomsheet 25 which might conceivably be in contact with the wearer if the backsheet 30 is removed. The tissue layers 18 and 19 are generally desirable since the airfelt layer 13 has little or no inherent strength, they reduce the tendency of the absorbent layer 13 to split, lump or ball when wetted, and they help to improve lateral wicking of the absorbed exudates, thereby providing a more even distribution of the exudates throughout the absorbent layer 13. The tissue layers in this preferred embodiment are coterminous with the absorbent layer 13, but they alternatively may have different dimensions or a different configuration. Tissues such as those previously described in relation to the bottomsheet 25, and others, are acceptable. The topsheet 15 overlays the first major surface 26 of the absorbent core 20 and extends beyond the core side edges 22 and the core end edges 23 to the longitudinal edges 33 and the end edges 34, respectively of the insert 10. The bottomsheet 25 overlays the second major surface 27 of the absorbent core 20 and extends beyond the core side edges 22 and the core end edges 23 to the longitudinal edges 33 and the end edges 34, respectively of the insert 10. The bottomsheet 25 and the topsheet 15 are affixed to one another near and along the edges 33 and 34 of the insert 10 by second attachment means. The second attachment means are nonfrangible lines of adhesive 81 and 82. In this embodiment, the bottomsheet 25 and the topsheet 15 are made of the same material. When the topsheet 15 and the bottomsheet 25 are made of the same material they may be two different sheets which are joined to each other (i.e., integral), as shown in Figures 4 and 5, or they may be a continuous and undivided unitary sheet (i.e., unitary) that wraps around and envelopes the absorbent core 20, the combination of these elements being superimposed on a backsheet 30. In this preferred embodiment, the backsheet 30 overlays the bottom surface 21 of the bottomsheet 25 and extends beyond the edges 22 and 23 on the absorbent core 20 to the edges 33 and 34, respectively, of the insert 10. The backsheet 30 is affixed to the bottomsheet 25 near and along the longitudinal edges 33 of the insert 10 by first attachment means. In this embodiment, the first attachement means are nonfrangible lines of adhesive 83 and 84.

In this embodiment shown in Figures 4 and 5, an alternatively preferred method for making the backsheet 30 removable from the remainder of the insert 10 is disclosed. The method involves making a lesser portion 45 of the backsheet 30 removable from the remainder of the backsheet 30 and the insert 10. The method involves making a longitudinal perforation 60, which is a series of cut-outs 61 and uncut bonds 62, in and through the backsheet 30 extending from one end edge 34 of the insert 10 to the other end edge 34 of the insert 10 near and along each longitudinal side edge 33 of the insert 10, as shown in Figure 4. The lesser portion 45 of the backsheet 30 is between the perforations 60. In this preferred embodiment, the backsheet 30 is affixed to the bottomsheet 25 between each perforation 60 and each perforation's 60 corresponding longitudinal edge 33 by the nonfrangible lines of adhesive 83 and 84. The lines of adhesive should have a great enough strength that their bond will not be broken when the lesser portion 45 of the backsheet 30 is removed (i.e., a greater tensile and shear strength than the perforations 60). The backsheet 30 is left unaffixed from the bottomsheet 25 between the perforations 60 so that the lesser portion 45 of the backsheet 30 can be easily torn away from the remainder of the insert 10. If the backsheet 30 is needed to be removed from the remainder of the insert 10, the user simply takes hold of the backsheet 30 between the perforations 60 and strips the lesser portion 45 of the backsheet 30 from the remainder of the backsheet 30. The remainder of the backsheet 30 remains affixed to the bottomsheet 25 by the nonfrangible lines of adhesive 83 and 84.

In this embodiment, the insert 10 further comprises a friction creating layer 53 and a pair of leg cuff members 50. A friction-creating layer 53 is joined to the backsheet 30 on the surface of the backsheet 30 furthest away from the absorbent core 20. The friction creating layer 53 is a strip of foam having a thickness of 3.0 millimeters (0.12 inch). Alternatively, the friction-creating layer 53 might be a pressure-sensitive adhesive or any other material which would help prevent the insert 10 from slipping or sliding when placed in the crotch portion of an undergarment. Further, in this preferred embodiment, leg cuff members 50 are joined to the insert 10 adjacent to the longitudinal edges 33 and are folded onto the backsheet 30 and topsheet 15. The leg cuff members 50 are made of a foam material and have a cross section of 6.5 centimeters (2.6 inches) by 0.3 centimeters (0.12 inch). The leg cuff members 50 are folded such that 4.5 centimeters (1.8 inches) of the member covers the topsheet and 2.0 centimeters (0.8 inch) of the member covers the backsheet 30. Each leg cuff member 50 is attached to the backsheet 30 along its entire length by attachment means 88 which are well known in the art. Portions of the leg cuff members 50 are also attached to the topsheet 15 near the end edges 34 of the insert 10 by attachment means (not shown) as are well known in the art. The remainder of each leg cuff member 50 is left unattached so that when the insert 10 is curved when placed in an undergarment or due to the natural contour of the wearer's lower torso, the leg cuff members 50 will bow slightly upwards and create containment pockets along the longitudinal edges 33 of the insert 10.

In another alternative embodiment of the insert 10 as shown in Figure 6, the backsheet 30 is not perforated from one end edge 34 of the insert 10 to the other end edge 34 of the insert 10, but has instead a continuously extending perforation 60 within the periphery 31 of the backsheet 30, wherein the lesser portion 45 of the backsheet 30 is bordered by the perforation 60. For example, a square, triangular, circular, rectangular, etc. portion 45 could be perforated within the periphery 31 of the backsheet 30, which portion 45 could then be easily torn away if necessary. The portion of the backsheet 30 outside or outboard of the perforation 60 is nonfrangibly affixed to the absorbent element 11 by second attachment means. Figure 6 is a bottom plan view of an alternatively preferred insert 10 in which a lesser portion 45 of the backsheet 30 has been perforated in a rectangular shape. The perforated lesser portion 45 has a pull tab 47 affixed to it to facilitate removal of the lesser portion 45.

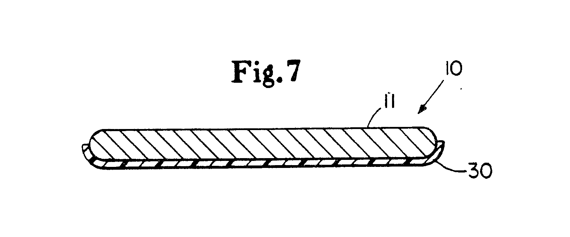

Another alternatively preferred insert embodiment of the present invention is shown by transverse cross-section in Figure 7. In this embodiment, the absorbent element 11 is a thermal formable substance. Suitable thermal formable substances include foam materials such as cross-linked polyolefin, cross-linked polyethylene, polypropylene, polybutylene, ethylene vinyl acetate, and polyurethane. Because of their own physical characteristics, such absorbent foam materials have structural integrity without the need for being encased between layers of other materials. The thermal formable substance must be absorbent and should be of an open cell type so that absorbed liquids can pass all the way through. A liquid impermeable backsheet 30 overlays the garment surface 27 of the absorbent element 11 and extends about half way up to the core side edges 22 and the core end edges 23 to provide containment of absorbed exudates. The backsheet 30 is removably affixed to the absorbent element 11 by a frangible attachment means (not shown) such as a pressure sensitive adhesive.

## Claims

1. An integral disposable absorbent article, said article having end edges and longitudinal edges and comprising:

   a) an absorbent element having structural integrity, and being formed within a body surface and a garment surface; and

   b) a liquid impermeable backsheet overlaying said garment surface of said absorbent element, said backsheet being removably affixed to said absorbent element, whereby the removal of said backsheet does not destroy the structural integrity of said absorbent element.

2. An integral disposble absorbent article as claimed in Claim 1, wherein said absorbent element comprises a thermally formable substance.

3. An integral disposable absorbent article as claimed in either one of Claims 1 and 2, wherein said absorbent element comprises an absorbent core and a liquid permeable topsheet, said absorbent core having a first major surface and a second major surface, said topsheet overlaying said first major surface of said absorbent core, and said backsheet overlaying said second major surface of said absorbent core.

4. An integral disposable absorbent article as claimed in Claim 3, wherein said absorbent element further comprises a liquid permeable bottomsheet having a bottom surface, said bottomsheet overlaying said second major surface of said absorbent core and said backsheet overlaying said bottom surface of said bottomsheet.

5. An integral disposal article as claimed in Claim 4, wherein said topsheet and said bottomsheet are integral.

6. An integral disposable absorbent article as claimed in either one of claims 4 and 5, wherein said absorbent core has core side edges, said topsheet, said bottomsheet, and said backsheet extending beyond said core side edges.

7. An integral disposable absorbent article as claimed in Claim 6, wherein said backsheet is affixed to said bottomsheet by first attachment means and said bottomsheet is affixed to said topsheet by second attachment means, said second attachment means being nonfrangible.

8. An integral disposable absorbent article as claimed in Claim 6, wherein said bottomsheet has bottomsheet side edges, said topsheet and said backsheet extend beyond said bottomsheet side edges, said backsheet is affixed to said topsheet by first attachment means and said bottomsheet is affixed to said topsheet by second attachment means, said second attachment means being nonfrangible.

9. An integral disposable absorbent article as claimed in Claim 4, wherein said topsheet and said bottomsheet are a unitary sheet, said backsheet being affixed to said unitary sheet by first attachment means.

10. An integral disposable absorbent article as claimed in any one of claims 7, 8 or 9, wherein said first attachment means is frangible.

11. An integral disposable absorbent article as claimed in any one of claims 7, 8 or 9, wherein said first attachment means is nonfrangible and said backsheet has a lesser portion, said lesser portion of said backsheet being removable from said article.

12. An integral disposable absorbent article as claimed in Claim 11, wherein said backsheet is perforated, said lesser portion of said backsheet being removable from said article by breaking said perforations.

13. An integral disposable absorbent article as claimed in Claim 12, said backsheet having a pair of longitudinally extending perforations each of which extends from one of said article end edges to the other of said article end edges, said first attachment means being located laterally outwardly of each of said perforations and laterally inwardly of the respective longitudinal article edges, said lesser portion of said backsheet being between said perforations.

14. An integral disposable absorbent article as claimed in Claim 12, wherein said perforation is continuous and borders said lesser portion of said backsheet, said first attachment means being outboard of said perforation.

15. An integral disposable absorbent article as claimed in any one of Claims 3-14, wherein said absorbent core has an airfelt layer.

16. An integral disposable absorbent article as claimed in Claim 15, wherein said absorbent core has a first tissue layer and a second tissue layer, said airfelt layer being disposed between said tissue layers.

17. An integral disposable absorbent article as claimed in any one of claims 4-16, wherein said bottomsheet has a dry tensile strength of greater than 70 grams per centimeter, preferably of greater than 160 grams per centimeter.

18. An integral disposable absorbent article as claimed in any one of the preceding claims further comprising a friction-creating layer, said friction-creating layer being adjacent to and joined to said backsheet on the surface of said backsheet furthest away from said absorbent element.

19. An integral disposable absorbent article as claimed in any one of the preceding claims further comprising a leg cuff member, said leg cuff member extending along said longitudinal edge of said article and being joined to said article.

# Fig. 1

# Fig.2

# Fig.3

# Fig. 4

10

5

53

50

45

34

30

33 60 62 61

5

50

# Fig. 5

10

26 13

20 18 15

81

22

22 82

83

84

50

33

50

88 60 27 25 21 30 53

19

60

88

# Fig. 6

10

45

30

47

34

60 62 61

31 33

# Fig.7